Europäisches Patentamt

European Patent Office

Office européen des brevets

(19)

(11) Publication number: **0 068 790**
**B1**

(12) **EUROPEAN PATENT SPECIFICATION**

(45) Date of publication of patent specification: **26.02.86**

(21) Application number: **82303238.8**

(22) Date of filing: **22.06.82**

(51) Int. Cl.⁴: **A 61 K 39/395,** C 07 K 15/00
// C12P1/00, C12R1/91,
C12N15/00

(54) Allele specific immunotherapeutic method and dosage form.

(30) Priority: **25.06.81 US 277398**
**22.09.81 US 304595**

(43) Date of publication of application:
**05.01.83 Bulletin 83/01**

(45) Publication of the grant of the patent:
**26.02.86 Bulletin 86/09**

(84) Designated Contracting States:
**AT BE CH DE FR GB IT LI LU NL SE**

(56) References cited:

CHEMICAL ABSTRACTS, vol. 92, no. 11, 17th
March 1980, page 446, no. 92582s, Columbus
Ohio (USA); D.J. CHARRON et al.: "Analysis of
HLA-D region-associated molecules with
monoclonal antibody"

NATURE, vol. 276, 23rd November 1978, pages
397-399, MacMillan Journals Ltd.; P. PARHAM
et al.: "Monoclonal antibody to a human
histocompatibility alloantigen, HLA-A2"

(73) Proprietor: **THE BOARD OF TRUSTEES OF THE
LELAND STANFORD JUNIOR UNIVERSITY**
**Office of Technology and Licensing 105 Encina
Hall Stanford University**
**Stanford California 94305 (US)**

(72) Inventor: **McDevitt, Hugh O.**
**618 Wildwood Lane**
**Palo Alto California 94303 (US)**

(74) Representative: **Harrison, David Christopher
et al**
**MEWBURN ELLIS & CO 2/3 Cursitor Street**
**London EC4A 1BQ (GB)**

Courier Press, Leamington Spa, England.

**0 068 790**

(56) References cited:

NATURE, vol. 290, no. 5807, 16th April 1981,
pages 591-593, MacMillan Journals Ltd.,
Chesham, Bucks (GB); L.M. NADLER et al.:
"Monoclonal antibody identifies a new Ia-like
(p29,34) polymorphic system linked to the
HLA-D/DR region"

NATURE, vol. 271, 26th January 1978, pages
375-377; D.P. ADEN et al.: "Production of HLA
antibody in mice immunised with syngeneic
mouse-human hybrid cells containing human
chromosome 6"

TISSUE ANTIGENS, vol. 16, 1980, pages 30-48;
F.M. BRODSKY et al.: "Monoclonal antibodies
to HLA-DRw determinants"

Description

The invention is in the field of immunotherapy. More specifically it relates to controlling diseases associated with a particular allele of certain regions the major histocompatibility complex (MHC), such as the HLA-D region in humans, by selectively suppressing the immune response(s) controlled by the allele with an allele-specific monoclonal antibody.

Immunosuppression is commonly achieved through treatment with a means and/or agent such as radiation, antimitotics, heterologous antilymphocyte sera, heretologous anti-T cell antibody, adrenal steroids, and cytotoxic chemicals. Such treatment is non-specific in the sense that it suppresses the entire immune system rather than a single immune response. The major side effect of nonspecific or general immunosuppression is immunodeficiency which leaves the treated individual highly susceptible to bacterial, viral, and fungal infections that would otherwise be manageable but under the circumstances are potentially life-threatening.

Antibodies are the most inherently specific natural immunosuppressive agents. Antibody regulation of the mouse immune system has been reported by Greene, et al, PNAS 74:5118—5121, 1977 and Meruelo, et al, PNAS 77:2178, 1980. Their experiments indicated that different anti-Ia sera could both increase and decrease tumor growth in vivo. This correlated with suppression or enhancement of the specific immune response. Antibody immunosuppressive therapy is used in certain instances in humans, such as for preventing Rh-related erythroblastosis fetalis. This mode of treatment, however, is limited to disease in which the offending antigen is known and a specific human antiserum is available.

Murine monoclonal antibodies have been developed against various antigens, including H-2 antigens. See, for instance Galfre, et al, *Antibodies to major histocompatibility antigens produced by hybrid cell lines*, Nature, Vol 266, 7 April 1977. Although these antibodies have been directed against a particular histocompatibility antigen, they are species specific only, and have not been specific enough to be effective in treating allele specific autoimmune diseases. In summary, current immunotherapy practice does not include a technique for treating genetically controlled, MHC associated autoimmune diseases via selective immunosuppression. The prime objective of the present invention is to provide such technique.

An aspect of the invention is a unit dosage form for treating an individual to control a disease associated with an MHC-linked immune response gene allele of the individual the individual being heterozygous for the gene, comprising a monoclonal antibody against the product of the allele combined with a pharmaceutically acceptable vehicle, the amount of the monoclonal antibody in the dosage form being sufficient to substantially and selectively suppress the immune response controlled by the allele.

The term "control" and conjugates thereof, that are used herein to describe the purpose and/or result of the treatment is intended to mean prophylaxis and/or therapy. Accordingly the invention may be used to prevent or alleviate a disease associated with a particular immune response allele of the MHC of the individual being treated.

It is known that all mammals and probably all vertebrates possess basically equivalent MHC systems and that immunse response genes are linked to the MHC. The invention may, therefore, be used to control immune response allele associated diseases in heterozygous vertebrate animals, particularly in mammals. It is expected that the immunotherapeutic method of the invention will be used primarily in humans, as well as in domestic, pet, and sport animals.

Associations between the human MHC, denoted the HLA, and susceptibility to diseases are reported on the HLA and Disease Registry which is kept and published in Copenhagen by Ryder and Svejgaard. Such associations are determined by comparing the HLA types of a diseased population with the HLA types of a healthy population. Many diseases are associated with more than one HLA specificity. Most associations have involved the D region of the HLA. Some are race specific. The association of a particular HLA allele with susceptibility to a disease is dominant. Studies to date have shown associations with an enormous variety of diseases, including most autoimmune diseases, some of which are listed below.

| | Racial group | HLA gene type | Antigen frequency | | Relative risk |
|---|---|---|---|---|---|
| | | | Patients | Controls | |
| **Joint disease** | | | | | |
| Ankylosing spondylitis | Caucasian | B27 | 89.8 | 8.0 | 87.8 |
| | Japanese | B27 | 66.7 | 0.0 | 305.7 |
| Reiter's disease | Caucasian | B27 | 78.2 | 8.4 | 35.9 |
| Yersinia arthritis | Caucasian | B27 | 79.4 | 9.4 | 24.3 |
| Salmonella arthritis | Caucasian | B27 | 66.7 | 8.6 | 17.6 |
| Psoriatic arthritis | Caucasian | B13 | 19.8 | 5.5 | 4.8 |
| Central Jones | | B27 | 40.2 | 8.7 | 8.6 |
| Juvenile rheumatoid arthritis | Caucasian | B27 | 26.4 | 8.5 | 4.7 |
| Rheumatoid arthritis | Caucasian | Dw4 | 42.2 | 15.7 | 3.0 |
| | Caucasian | Dw3 | 30.0 | 17.0 | 2.7 |
| Sjogren's syndrome | Caucasian | B8 | 50.6 | 24.0 | 3.2 |
| | | Dw3 | 53.5 | 17.0 | 5.2 |
| **Neurological disease** | | | | | |
| Multiple sclerosis | Caucasian | Dw2 | 60.0 | 15.0 | 8.5 |
| Myasthenia gravis | Caucasian | A1 | 44.6 | 26.3 | 2.45 |
| | Caucasian | B8 | 57.7 | 22.9 | 4.40 |
| | Caucasian | Dw3 | 43.0 | 17.0 | 3.5 |
| Paralytic poliomyelitis | Caucasian | B7 | 37.8 | 19.0 | 2.6 |
| **Endocrine glands** | | | | | |
| Graves' disease | Caucasian | B8 | 36.7 | 21.7 | 2.13 |
| **Thyroid** | | Dw3 | 50.0 | 21.7 | 4.0 |
| | Japanese | Bw35 | 56.8 | 20.5 | 4.97 |
| deQuervain's thyroiditis | Caucasian | Bw35 | 76.9 | 12.9 | 22.2 |
| **Adrenal** | | | | | |
| Addison's disease | Caucasian | B8 | 50.0 | 22.7 | 3.9 |
| | | Dw3 | 70.0 | 21.7 | 10.5 |
| **Pancreas** | | | | | |
| Diabetes | Caucasian | B8 | 36.7 | 21.8 | 2.1 |
| Juvenile onset diabetes | | Bw15 | 22.8 | 14.9 | 2.1 |
| | | Dw3 | 50.0 | 4.7 | 4.5 |
| **Gastrointestinal tract** | | | | | |
| Chronic active hepatitis | Caucasian | A1 | 41.6 | 28.4 | 1.8 |
| | | B8 | 44.2 | 20.3 | 3.0 |
| | | Dw3 | 60.0 | 21.7 | 7.2 |

# 0 068 790

|  | Racial group | HLA gene type | Antigen frequency | | |
|---|---|---|---|---|---|
|  |  |  | Patients | Controls | Relative risk |
| **Joint disease** | | | | | |
| Celiac disease (gluten enteropathy) | Caucasian | A1 | 63.7 | 29.5 | 4.2 |
|  |  | B8 | 71.2 | 23.1 | 8.8 |
|  |  | Dw3 | 98.0 | 15.0 | 8.8 |
|  |  | Dw3 | 98.0 | 15.0 | 278. |
| Hemochromatosis | Caucasian | A3 | 78.4 | 27.0 | 9.5 |
|  |  | B14 | 25.5 | 3.4 | 9.23 |
| Ulcerative colitis | Japanese | B5 | 80.0 | 30.8 | 9.3 |
| Pernicious anemia | Caucasian | B7 | 35.8 | 22.1 | 2.2 |
| **Skin** | | | | | |
| Psoriasis | Caucasian | B13 | 19.7 | 4.5 | 4.65 |
|  |  | Bw17 | 26.2 | 7.8 | 4.7 |
|  |  | Bw37 | 7.7 | 1.4 | 5.4 |
|  |  | D-LD MA | 36.0 | 5.0 | 10.2 |
| Dermatitis herpetiformis | Caucasian | A1 | 69.0 | 30.1 | 4.4 |
|  |  | B8 | 77.0 | 24.7 | 9.2 |
| Pemphigus vulgaris | Caucasian | A10 | 39.3 | 12.7 | 3.1 |
| Bechet's disease | Caucasian | B5 | 35.1 | 11.1 | 4.3 |
|  | Japanese | B5 | 75.0 | 30.8 | 6.5 |
| Herpes labialis | Caucasian | A1 | 55.6 | 25.1 | 3.7 |
|  |  | B8 | 33.3 | 16.8 | 2.5 |
| **Eye disease** | | | | | |
| Anterior uvetis | Caucasian | B27 | 56.8 | 7.7 | 15.4 |
| Vogt-Koyanagi-Harada disease | Japanese | B22J | 42.9 | 13.2 | 4.5 |
|  |  | D-LD YT | 66.6 | 16.0 | 11.2 |
| **Congenital malformation** | | | | | |
| Congenital heart disease | Caucasian | A2 | 80.0 | 43.9 | 4.9 |

Preferably the invention is used to control diseases having a high degree of association with one or more particular HLA gene types. Examples of such associations are: B27—ankylosing spondylitis; Dw4—rheumatoid arthritis and juvenile onset diabetes; Dw3—Sjogren's syndrome, myasthenia gravis, Addison's disease, juveline onset diabetes, chronic active hepatitis, and celiac disease; and Dw2—multiple sclerosis.

In order for immunotherapy to be specifically directed against the immune response controlled by the allele associated with the disease, the individual must be heretozygous for the allele. Otherwise the response of the other (identical) allele at the locus would quite likely be suppressed as well. The heterozygosity of an individual for a particular allele may be established by standard HLA typing methods such as typing by cytotoxicity using pregnancy sera or by mixed lymphocyte reaction (HLA-D) using cells from individuals that are homozygous for the HLA region involved. Most individuals (80%—90%) are heterozygous for HLA—A,B,C and D.

According to the invention, diseases are controlled by selective immunosuppression using monoclonal antibodies having a specific affinity for the product of the particular responder allele associated with the disease. In instances in which the disease is associated in different populations with more than one allele, it may be desirable to administer monoclonal antibodies against each of the associated allele products concurrently or sequentially. Recent evidence indicates that immune response gene products are "Ia" molecules- -surface glycoproteins composed of α and β chains. Monoclonal antibodies directed against such products may be made from antibody expressing hybridomas by well known

5

procedures such as those described by Kohler, G. and Milstein, C. (1975) Nature 356: 497—7; Levy, R. and Dilley, I. (1978) PNAS USA 75: 4211—4215; Galfre et al, supra; and Ollson, C. and Kaplan, H. (1980) PNAS USA 77: 5429—5431. Briefly, these processes involve fusing myeloma cells and lymphocytes in a nonionic detergent medium, typically polyethylene glycol. The fused cells or hybridomas are then expanded in a nutrient medium and then selected by incubation in a selective medium such as HAT medium. The cells surviving the incubation are assayed for production of the desired antibody and positive cells are sorted and cloned by known techniques. The monoclonal antibodies expressed by the clones may be harvested and purified by known techniques. Myeloma cell lines that may be used in the process are known and available. The lymphocytes, typically either spleen cells or B-cells, are obtained from immunized individuals known to have a high titer of the desired anti-MHC antibody. Pregnancy sera is a good source for such B-cells. In this manner monoclonal antibodies that are directed specifically against the product of the particular allele associated with the disease to be treated may be prepared. Examples of antibodies that may be made and used in the invention are anti-HLA-B27 monoclonal antibody, anti-HLA-Dw2 monoclonal antibody, anti-HLA-Dw3 monoclonal antibody, and anti-Dw4 monoclonal antibody.

Although xenogeneic antibodies may be used in the invention, it is preferable to use allogeneic antibodies to reduce the likelihood of the antibodies themselves inducing an immune response from the host. An allogeneic monoclonal antibody is one that is expressed by a hybridoma made by fusing cells from the same animal species as the host. The antibodies may be from one or more immunoglobulin classes (IgM, IgG, IgA, IgD, or IgE) depending upon the particular disease and individual involved. Antigen binding fragments (F(ab')$_2$, Fab, Fab') of IgG monoclonal antibodies may also be used in appropriate situations, for instance, where it is desired to reduce the likelihood of complement fixation. As used herein, the term "monoclonal antibody" is intended to include such fragments as well as whole immunoglobulins.

The anti-MHC allele monoclonal antibodies are preferably administered to the individuals in a manner that will maximize the likelihood of the antibody reaching the products of the disease associated allele, binding to those products, and thereby blocking the immune response that leads to or perpetuates the disease. Being proteins, the antibodies will normally be administered parenterally, preferably intravenously. Since they may react with white blood cells, they will preferably be administered slowly either from a conventional IV administration set or from a subcutaneous depot. Murine tests indicate that the amount of antibody required to suppress an immune response will typically be in the range of 1 to 3 g/l of blood volume, more usually 1.5 to 2.5

g/l of blood volume. This dosing may have to be repeated periodically depending upon the particular disease. When used as a prophylaxis it may be possible to administer the antibody or antibodies semiannually or annually. In treating an existing disease it is expected that the antibody or antibodies will be administered more frequently as needed. For autoimmune diseases that are known to be triggered or aggravated by particular environmental factors, the dosage regimen will be scheduled accordingly.

When administered parenterally the antibodies will be formulated in a unit dosage injectable form (solution, suspension, emulsion) in association with a pharmaceutically acceptable parenteral vehicle. Such vehicles are inherently nontoxic and nontherapeutic. Examples of such vehicles are water, saline, Ringer's solution, dextrose solution, and Hank's solution. Nonaqueous vehicles such as fixed oils and ethyl oleate may also be used. A preferred vehicle is 5w% dextrose/saline. The vehicle may contain minor amounts of additives such as substances that enhance isotonicity and chemical stability, e.g. buffers and preservatives. The antibody is preferably formulated in purified form substantially free of aggregates and other proteins at concentrations of about 5 to 30 mg/ml, preferably 10 to 20 mg/ml.

The following examples further illustrate the invention. These examples are not intended to limit the invention in any manner.

Example 1

H-2$^{k/b}$ mice are heterozygous for the H-2$^k$ (I-A$^k$) and H-2$^b$ (I-A$^b$) alleles, H-2$^k$ mice respond to (H,G)-A--L but not to (T,G)-A--L. H-2$^b$ mice respond to (T,G)-A--L but not (H,G)-A--L. The H-2$^{k/b}$F$_1$ heterozygote responds to both antigens. Treating these F$_1$ mice with two ml of a 5—10 mg/ml solution of an anti I-A$^k$ monoclonal antibody, suppresses the immune response to two aqueous injections of (H,G)-A--L but not to (T,G)-A--L. This is thus allele specific immunosuppression for an antigen for which the high responder is I-A$^k$ and not I-A$^b$.

Example 2

One mouse autoimmune disease, EAE, has been suppressed in the homozygous H-2$^{s/s}$ mouse using monoclonal anti-I-A$^s$.

In view of the known correlations between the chemistry and function of the murine H-2 system and its gene products and the human HLA system and its gene products, persons of skill in immunology will find the murine responses to anti-H-2 allele monoclonal antibodies reported above convincing evidence that humans will respond similarly to anti-HLA allele monoclonal antibodies.

Example 3

This Example provides a hypothetical protocol for confirming the efficacy of the invention process in controlling multiple sclerosis in humans. The patient population is 50—100 adult D type heterozygous persons who are HLA-Dw2 positive and have rapidly progressive or non-remitting multiple sclerosis. One half of this population is treated with human anti-HLA-Dw2 monoclonal antibody at a dose of 2 g/l of blood volume administered intravenously over several hours. This treatment is repeated weekly for four months. The other half of the population is treated in the same manner but with an innocuous comparable control monoclonal antibody that is not directed against the patients' D type. A comparison of the efficacy of this treatment against the control based on evaluation of the patients' neurological lesions shows the efficacy of the anti-HLA-Dw2 monoclonal antibody treatment in controlling multiple sclerosis.

Modifications of the above modes for carrying out the invention that are obvious to those of skill in related arts, e.g. molecular biology, biochemistry, immunology, and medicine, are intended to be within the scope of the following claims.

**Claims**

1. A unit dosage form for treating an individual to control a disease associated with an MHC-linked immune response gene allele of the individual, the individual being heterozygous for the gene, comprising a monoclonal antibody against the product of the allele combined with a pharmaceutically acceptable vehicle, the amount of the monoclonal antibody in the dosage form being sufficient to substantially and selectively suppress the immune responses controlled by the allele.

2. The unit dosage form of Claim 1 wherein the monoclonal antibody is an allogeneic monoclonal antibody.

3. The unit dosage form of Claim 1 or 2 wherein the individual is a human, the disease is multiple sclerosis, the allele is HLA-Dw2 and the monoclonal antibody is anti-HLA-Dw2.

4. The unit dosage form of Claim 1 or 2 wherein the individual is a human, the disease is myasthenia gravis, the allele is HLA-Dw3, and the monoclonal antibody is anti-HLA-Dw3.

5. The unit dosage form of Claim 1 or 2 wherein the individual is a human, the disease is rheumatoid arthritis, the allele is at least one of HLA-Dw4 and HLA-Dw3, and the monoclonal antibody is correspondingly at least one of anti-HLA-Dw4 and anti-HLA-Dw3.

6. The unit dosage form of Claim 1 or 2 wherein the individual is human, the disease is juvenile onset diabetes, the allele is at least one of HLA-Dw4 and HLA-Dw3, and the monoclonal antibody is correspondingly at least one of anti-HLA-Dw4 and anti-HLA-Dw3.

7. The unit dosage form of Claim 1 or 2 wherein the dosage form is a parenteral dosage form.

8. The unit dosage form of Claim 1 or 2 wherein the dosage form is an intravenous dosage form, the vehicle is a solvent for the antibody, and the concentration of the antibody in the dosage form is about 5 to 30 mg/ml.

9. The unit dosage form of Claim 7 or 8 wherein said amount of monoclonal antibody is in the range of 1 to 3 g per liter of blood volume of the individual.

10. Monoclonal antibody anti-HLA-Dw2 for use in the treatment of multiple sclerosis in individuals with a gene heterozygous for the relevant immune response.

11. Monoclonal antibody anti-HLA-Dw3 for use in the treatment of myasthenia in individuals with a gene heterozygous for the relevant immune response.

12. Monoclonal antibody anti-HLA-Dw4 or anti-HLA-Dw3 for use in the treatment of rheumatoid arthritis in individuals with a gene heterozygous for the relevant immune response.

13. Monoclonal antibody anti-HLA-Dw4 or anti-HLA-Dw3 for use in the treatment of juvenile onset diabetes in individuals with a gene heterozygous for the relevant immune response.

**Claims for the Contracting State: AT**

1. A method of preparing a unit dosage form for treating an individual to control a disease associated with an MHC-linked immune response gene allele of the individual, the individual being heterozygous for the gene, comprising using a monoclonal antibody against the product of the allele combined with a pharmaceutically acceptable vehicle, the amount of the monoclonal antibody in the dosage form being sufficient to substantially and selectively suppress the immune responses controlled by the allele.

2. The method of Claim 1 wherein the monoclonal antibody is an allogeneic monoclonal antibody.

3. The method of Claim 1 or 2 wherein the individual is a human, the disease is multiple sclerosis, the allele is HLA-Dw2 and the monoclonal antibody is anti-HLA-Dw2.

4. The method of Claim 1 or 2 wherein the individual is a human, the disease is myasthenia gravis, the allele is HLA-Dw3, and the monoclonal antibody is anti-HLA-Dw3.

5. The method of Claim 1 or 2 wherein the individual is a human, the disease is rheumatoid arthritis, the allele is at least one of HLA-Dw4 and HLA-Dw3, and the monoclonal antibody is correspondingly at least one of anti-HLA-Dw4 and anti-HLA-Dw3.

6. The method of Claim 1 or 2 wherein the individual is a human, the disease is juvenile onset diabetes, the allele is at least one of HLA-Dw4 and HLA-Dw3, and the monoclonal antibody is correspondingly at least one of anti-HLA-Dw4 and anti-HLA-Dw3.

7. The method of Claim 1 or 2 wherein the dosage form is a parenteral dosage form.

8. The method of Claim 1 or 2 wherein the dosage form is an intravenous dosage form, the vehicle is a solvent for the antibody, and the

9　0 068 790　10

concentration of the antibody in the dosage form is about 5 to 30 mg/ml.

9. The method of Claim 7 or 8 wherein said amount of monoclonal antibody is in the range of 1 to 3 per liter of blood volume of the individual.

**Revendications pour les Etats Contractants: BE CH DE FR GB IT LI LU NL SE**

1. Forme de dosage unitaire pour le traitement d'un individu pour contrôler une maladie associée à un allèle génétique à immunoréponse liée à MHC d'un individu, l'individu étant hétérozygote pour le gène, comprenant un anticorps monoclonal contre le produit de l'allèle combiné à un véhicule acceptable en pharmacie, la quantité de l'anticorps monoclonal dans la forme de dosage étant suffisante pour supprimer sensiblement et sélectivement les immunoréponses contrôlées par l'allèle.

2. Forme de dosage unitaire selon la revendication 1 où l'anticorps monoclonal est un anticorps monoclonal allogénéique.

3. Forme de dosage unitaire selon la revendication 1 ou 2 où l'individu est un être humain, la maladie est une sclérose en plaques, l'allèle est HLA-Dw2 et l'anticorps monoclonal ets anti-HLA-Dw2.

4. Forme de dosage unitaire selon la revendication 1 ou 2 où l'individu est un être humain, la maladie est la myasthénie grave, l'allèle est HLA-Dw3 et l'anticorps monoclonal est anti-HLA-Dw3.

5. Forme de dosage unitaire selon la revendication 1 ou 2 où l'individu est un être humain, la maladie est l'arthrite rhumatoïde, l'allèle est au moins l'un parmi HLA-Dw4 et HLA-Dw3, et l'anticorps monoclonal est de manière correspondante au moins l'un parmi anti-HLA-Dw4 et anti-HLA-Dw3.

6. Forme de dosage unitaire selon la revendication 1 ou 2 où l'individu est un être humain, la maladie est le diabète juvénile, l'allèle est au moins l'un parmi HLA-Dw4 et HLA-Dw3 et l'anticorps monoclonal est de manière correspondante au moins l'un parmi anti-HLA-Dw4 et anti-HLA-Dw3.

7. Forme de dosage unitaire selon la revendication 1 ou 2 où la forme de dosage est une forme de dosage parentéral.

8. Forme de dosage unitaire selon la revendication 1 ou 2 où la forme de dosage est une forme de dosage intraveineux, le véhicule est un solvant de l'anticorps, et la concentration de l'anticorps dans la forme de dosage est d'environ 5 à 30 mg/ml.

9. Forme de dosage unitaire selon la revendication 7 ou 8 où ladite quantité de l'anticorps monoclonal est comprise entre 1 et 3 g par litre de volume de sang de l'individu.

10. Anticorps monoclonal anti-HLA-Dw2 à utiliser pour le traitement de la sclérose en plaques chez des individus ayant un gène hétérozygote pour l'immunoréponse en rapport.

11. Anticorps monoclonal anti-HLA-Dw3 à utiliser pour le traitement de la myasthénie chez des individus ayant un gène hétérozygote pour l'immuno-réponse en rapport.

12. Anticorps monoclonal anti-HLA-Dw4 ou anti-HLA-Dw3 à utiliser pour le traitement de l'arthrite rhumatoïde chez des individus ayant un gène hétérozygote pour l'immunoréponse en rapport.

13. Anticorps monoclonal anti-HLA-Dw4 ou anti-HLA-Dw3 à utiliser pour le traitement du diabète juvénile chez des individus ayant un gène hétérozygote pour l'immuno-réponse en rapport.

**Revendications pour l'Etat Contractant: AT**

1. Procédé d'obtention d'un forme de dosage unitaire pour le traitement d'un individu pour contrôler une maladie associée à un allèle génétique à immuno-réponse lié à MHC d'un individu, l'individu étant hétérozygote pour le gène, comprenant l'utilisation d'un anticorps monoclonal contre le produit de l'allèle combiné à un véhicule acceptable en pharmacie, la quantité de l'anticorps monoclonal dans la forme de dosage étant suffisante pour supprimer sensiblement et sélectivement les immuno-réponses contrôlées par l'allèle.

2. Procédé selon la revendication 1 où l'anticorps monoclonal est un anticorps monoclonal allogénéique.

3. Procédé selon la revendication 1 ou 2 où l'individu est un être humain, la maladie est une sclérose en plaques, l'allèle est HLA-Dw2 et l'anticorps monoclonal est anti-HLA-Dw2.

4. Procédé selon la revendication 1 ou 2 où l'individu est un être humain, la maladie est la myasthénie grave, l'allèle est HLA-Dw3 et l'anticorps monoclonal est anti-HLA-Dw3.

5. Procédé selon la revendication 1 ou 2 où l'individu est un être humain, la maladie est l'arthrite rhumatoïde, l'allèle est au moins l'un parmi HLA-Dw4 et HLA-Dw3, et l'anticorps monoclonal est de façon correspondante au moins l'un parmi anti-HLA-Dw4 et anti-HLA-Dw3.

6. Procédé selon la revendication 1 ou 2 où l'individu est un être humain, la maladie est le diabète juvénile, l'allèle est au moins l'un parmi HLA-Dw4 et HLA-Dw3, et l'anticorps monoclonal est de façon correspondate au moins l'un parmi anti-HLA-Dw4 et anti-HLA-Dw3.

7. Procédé selon la revendication 1 ou 2 où la forme de dosage est une forme de dosage patentéral.

8. Procédé selon la revendication 1 ou 2 où la forme de dosage est une forme de dosage intraveineux, le véhicule est un solvant de l'anticorps, et la concentration de l'anticorps dans la forme de dosage est d'environ 5 à 30 mg/ml.

9. Procédé selon la revendication 7 ou 8 où ladite quantité de l'anticorps monoclonal est comprise entre 1 et 3 g par litre de volume de sang de l'individu.

## Patentansprüche für die Vertragsstaaten: BE CH DE FR GB IT LI LU NL SE

1. Eine Einheitsverabreichungsform für die Behandlung eines Individuums zur Bekämpfung einer Krankheit, die mit einem MHC-gebundenen Immunreaktionsgenallel des Individuums assoziiert ist, wobei das Individuum heterozygot für das Gen ist, umfassend einen monoklonalen Antikörper gegen das Produkt des Allels kombiniert mit einem pharmazeutisch verträglichen Trägermittel, die Menge an monokanalem Antikörper in der Verabreichungsform ausreichend für die wesentliche und selektive Underdrückung der vom Allel gesteuerten Immunreaktion.

2. Die Einheitsverabreichungsform nach Anspruch 1, worin der monoklonale Antikörper ein allogener, monoklonaler Antikörper ist.

3. Die Einheitsverabreichungsform nach Anspruch 1 oder 2, worin das Individuum ein Mensch ist, die Krankheit multiple Sklerose ist, das Allel HLA-Dw2 und der monoklonale Antikörper Anti-HLA-Dw2 ist.

4. Die Einheitsverabreichungsform nach Anspruch 1 oder 2, worin das Individuum ein Mensch, die Krankheit Myasthenia gravis, das Allel HLA-Dw3 und der monoklonale Antikörper Anti-HLA-Dw3 ist.

5. Die Einheitsverabreichungsform nach Anspruch 1 oder 2, worin das Individuum ein Mensch, die Krankheit rheumatoide Arthritis, das Allel wenigstens eines von HLA-Dw4 und HLA-Dw3 ist und der monoklonale Antikörper entsprechend wenigstens einer von Anti-HLA-Dw4 und Anti-HLA-Dw3 ist.

6. Die Einheitsverabreichungsform nach Anspruch 1 oder 2, worin das Individuum ein Mensch, die Krankheit Jugend-Diabetes, das Allel wenigstens eines von HLA-Dw4 und HLA-Dw3 ist und der monoklonale Antikörper wenigstens einer von Anti-HLA-Dw4 und Anti-HLA-Dw3 ist.

7. Die Einheitsverabreichungsform nach Anspruch 1 oder 2, worin die Verabreichungsform eine parenterale Verabreichungsform ist.

8. Die Einheitsverabreichungsform nach Anspruch 1 oder 2, worin die Verabreichungsform eine intravenöse Verabreichungsform ist, das Trägermittel ein Lösungsmittel für den Antikörper ist und die Konzentration des Antikörpers in der Verabreichungsform etwa 5—30 mg/ml ist.

9. Die Einheitsverabreichungsform nach Anspruch 7 oder 8, worin die genannte Menge an monoklonalem Antikörper innerhalb eines Bereiches von 1—3 g/l Blutvolumen des Individuums liegt.

10. Monokionaler Antikörper Anti-HLA-Dw2 für die Verwendung bei der Behandlung von multipler Sklerose bei Individuen mit einem Gen, das heterozygot für die relevante Immunreaktion ist.

11. Monoklonaler Antikörper Anti-HLA-Dw3 für die Verwendung bei der Behandlung von Myasthenie bei Individuen mit einem Gen, das heterozygot für die relevante Immunreaktion ist.

12. Monoklonaler Antikörper Anti-HLA-Dw4 oder Anti-HLA-Dw3 für die Verwendung bei der Behandlung von rheumatoider Arthritis bei Individuen mit einem Gen, das heterozygot für die relevante Immunreaktion ist.

13. Monoklonaler Antikörper Anti-HLA-Dw4 oder Anti-HLA-Dw3 für die Verwendung bei der Behandlung von Jugend-Diabetes bei Individuen mit einem Gen, das heterozygot für die relevante Immunreaktion ist.

## Patentansprüche für den Vertragsstatt: AT

1. Ein Verfahren zur Herstellung eine Einheitsverabreichungsform zur Behandlung eines Individuums zur Bekämpfung einer Krankheit, die mit einem MHC-gebundenen Immunreaktionsgenallel des Individuums assoziiert ist, wobei das Individuum heterozygot für das Gen ist, umfassend die Anwendung eines monoklonalen Antikörpers gegen das Produkt des Allels in Kombination mit einem pharmazeutisch verträglichen Trägermittel, wobei die Menge an monoklonalem Antikörper in der Verabreichungsform ausreicht zur wesentlichen und selektiven Unterdrückung der vom· Allel gesteuerten Immunreaktion.

2. Das verfahren nach Anspruch 1, worin der monoklonale antikörper ein allogener, monoklonaler Antikörper ist.

3. Das Verfahren nach Anspruch 1 oder 2, worin das Individuum ein Mensch, die Krankheit multiple Sklerose, das Allel HLA-Dw2 und der monoklonale Antikörper. Anti-HLA-Dw2 ist.

4. Das Verfahren nach Anspruch 1 oder 2, worin das Individuum ein Mensch, die Krankheit Myasthenia gravis, das Allel HLA-Dw3 und der monoklonale Antikörper Anti-HLA-Dw3 ist.

5. Das Verfahren nach Anspruch 1 oder 2, worin das Individuum ein Mensch, die Krankheit rheumatoide Arthritis, das Allel wenigstens eines von HLA-Dw4 und HLA-Dw3 ist und der monoklonale Antikörper entsprechend wenigstens einer von Anti-HLA-Dw4 und Anti-HLA-Dw3 ist.

6. Das Verfahren nach Anspruch 1 oder 2, worin das Individuum ein Mensch, die Krankheit Jugend-Diabetes, das Allel wenigstehs eines von HLA-Dw4 und HLA-Dw3 ist und der monoklonale Antikörper wenigstens einer von Anti-HLA-Dw4 und Anti-HLA-Dw3 ist.

7. Das Verfahren nach Anspruch 1 oder 2, worin die Verabreichungsform eine parenterale Verabreichungsform ist.

8. Das Verfahren nach Anspruch 1 oder 2, worin die Verabreichungsform eine intravenöse Verabreichungsform ist, das Trägermittel ein Lösungsmittel für den Antikörper ist und die Konzentration des Antikörpers in der Verabreichungsform etwa 5—30 mg/ml ist.

9. Das Verfahren nach Anspruch 7 oder 8, worin die genannte Menge an monoklonalem Antikörper innerhalb eines Bereiches von 1—3 g/l Blutvolumen des Individuums liegt.